# EUROPÄISCHE PATENTSCHRIFT

(11) **EP 1 144 311 B1**
(45) Veröffentlichungstag und Bekanntmachung des Hinweises auf die Patenterteilung: **16.04.2003**
(21) Anmeldenummer: 99931221.8
(22) Anmeldetag: 30.06.1999
(51) Int. Cl.: C01F 1/00

(54) **VERFAHREN ZUR ABREICHERUNG DES BROMIDGEHALTES AUS EINER WÄSSRIGEN BROMID ENTHALTENDEN LÖSUNG UNTER VERWENDUNG VON WASSERSTOFFPEROXID**
METHOD FOR REDUCING THE BROMIDE CONTENT IN AN AQUEOUS BROMIDE-CONTAINING SOLUTION USING HYDROGEN PEROXIDE
PROCEDE PERMETTANT D'APPAUVRIR LA TENEUR EN BROMURE D'UNE SOLUTION AQUEUSE CONTENANT DU BROMURE A L'AIDE D'EAU OXYGENEE

(30) Priorität: 07.07.1998 DE 19830310
(43) Veröffentlichungstag der Anmeldung: 17.10.2001
(73) Patentinhaber: Riedel-de Haen GmbH, 30926 Seelze (DE)
(72) Erfinder: REMMERS, Graalf, D-30823 Garbsen (DE); LIEKER, Horst, D-30165 Hannover (DE)
(74) Vertreter: Ricker, Mathias, Dr.Dipl.-Chem.
(86) Internationale Anmeldenummer: EP9904515
(87) Internationale Veröffentlichungsnummer: WO00002815

(56) Entgegenhaltungen:
- DE-A- 2 509 517
- US-A- 4 041 154
- "Europäisches Arzneibuch, 3. Ausgabe" 1997 , DEUTSCHER APOTHEKER VERLAG , STUTTGART XP002118311 in der Anmeldung erwähnt Seite 1234, Spalte 2 -Seite 1235, Spalte 1
- "Handbook of fine chemicals 1996-1997" 1996 , ALDRICH CATALOGUE XP002118312 Seite 941, Produkt Nummer 25,577-7 : Magnesium Chloride Hexahydrate, 99.995%

## Beschreibung

Die vorliegende Erfindung betrifft ein Verfahren zur Abreicherung des Bromidgehaltes einer Lösung unter Verwendung von Wasserstoffperoxid. Ebenso betrifft die vorliegende Erfindung ein Verfahren, in dem neben dem Bromidgehalt auch der Bromatgehalt abgereichert wird.

In vielen Anwendungsgebieten beispielsweise der Chemie oder der Pharmazie ist der maximale Bromidgehalt von verwendeten chemischen Verbindungen problematisch. Wichtig ist die Einhaltung von Höchstgrenzen der Bromidkonzentration u.a. in der pharmazeutischen Industrie.

Bei Verwendung der Bromid enthaltenden Chemikalien im pharmazeutischen Sektor werden die Reinheitsanforderungen der entsprechenden Pharmakopöen (Pharma Euro III, S. 1234-1235) zugrunde gelegt. Verbindungen, die in diesem Zusammenhang beispielsweise von Bedeutung sind, sind Alkali- und Erdalkaliverbindungen.

Insbesondere bei Alkali- und Erdalkaliverbindungen, die natürlichen Ursprungs sind, stellt der Bromidgehalt ein wesentliches Problem dar, denn zahlreiche Anionen von beispielsweise Alkali- und Erdalkalisalzen sind mit Bromid vergesellschaftet. Beispielsweise trifft dies auf natürlich vorkommende Alkali- und Erdalkalichloride zu.

Für Magnesiumchlorid fordert beispielsweise das Europäische Arzneibuch seit dem 1. Januar 1997 einen Grenzwert des Bromidgehaltes von 500 ppm.

Ein Verfahren zur Reduzierung des Bromidgehaltes, mit dem ein solcher Gesamtbromgehalt einer Verbindung zu erreichen ist, stellt beispielsweise das Einleiten von gasförmigem Chlor in eine Lösung der entsprechenden Verbindung in einem geeigneten Lösungsmittel, beispielsweise Wasser, dar.

Die DE-A 21 18 623 beispielsweise offenbart ein Verfahren zur Reinigung von wäßrigen Magnesiumchloridlösungen, in denen der Bromidgehalt durch Einleiten von Chlorgas in diese Lösungen reduziert wird.

In der DE-A 26 13 28 ist ein Verfahren zur Herstellung von hochkonzentrierten Magnesiumchloridlösungen beschrieben. Die in dieser Schrift geschilderte Entbromung findet mittels gasförmigen Chlors in der Wärme statt.

Der Umgang mit gasförmigem Chlor stellt aufgrund der Reaktivität dieses Gases jedoch ein hohes Gefahrenpotential dar. Demgemäß ist für die entsprechenden Verfahren bzw. Versuchsaufbauten aus Sicherheitsaspekten im allgemeinen ein hoher apparativer und folglich auch hoher finanzieller Aufwand erforderlich.

Eine Aufgabe der vorliegenden Erfindungen war es daher, ein apparativ einfach zu führendes technisches Verfahren bereitzustellen, in dem Bromid aus Lösungen abgereichert werden kann.

Gelöst wurde diese Aufgabe durch den Einsatz von Wasserstoffperoxid als Oxidationsmittel für Bromid. Demgemäß betrifft die vorliegende Erfindung ein Verfahren zur Abreicherung des Bromidgehaltes einer Lösung unter Verwendung von Wasserstoffperoxid.

Zu den wichtigsten Verbindungen, deren Lösungen zur Abreicherung des Bromidgehaltes durch das erfindungsgemäße Verfahren behandelt werden, zählen Alkali- und Erdalkaliverbindungen.

Daher betrifft die vorliegende Erfindung auch ein Verfahren, das dadurch gekennzeichnet ist, daß es sich bei der wäßrigen Lösung um eine Lösung von Alkali- und/oder Erdalkaliverbindungen handelt.

Insbesondere betrifft die vorliegende Erfindung ein Verfahren, das dadurch gekennzeichnet ist, daß es sich bei den Alkali- und Erdalkaliverbindungen um Alkali- und Erdalkalichloride handelt.

Im allgemeinen ist es im erfindungsgemäßen Verfahren unkritisch, in welcher Konzentration Wasserstoffperoxid eingesetzt wird. Insbesondere ist es möglich, diese Konzentration an die Erfordernisse der Reinigung anzupassen. Als solche sind beispielsweise die Verunreinigung der Lösung, d.h. deren Bromidgehalt oder auch deren Gehalt an weiteren, durch Wasserstoffperoxid oxidierbare Verunreinigungen zu nennen.

Der pH-Wert der Lösung, deren Bromidgehalt abgereichert werden soll, liegt im erfindungsgemäßen Verfahren im allgemeinen im Bereich < 7, bevorzugt im Bereich ≤ 6, und besonders bevorzugt im Bereich ≤ 5.

Daher betrifft die vorliegende Erfindung ein Verfahren, wie oben beschrieben, das dadurch gekennzeichnet ist, daß die wäßrige Lösung bei der Abreicherung einen pH-Wert von < 5 aufweist.

Der pH-Wert der Lösung wird im erfindungsgemäßen Verfahren, je nach Ausgangswert des pH der Lösung, über Säuren oder Basen auf den gewünschten Wert eingestellt.

Im allgemeinen ist die Zugabe von Säuren nötig, um den pH auf die gewünschten Werte, wie oben beschrieben, zu bringen. Als Säuren werden bevorzugt anorganische Säuren verwendet. Besonders bevorzugt ist hierbei Salzsäure.

Selbstverständlich ist es im Rahmen des erfindungsgemäßen Verfahrens möglich, die Säure, falls erforderlich, in gasförmigem Zustand der Lösung zuzugeben. Bevorzugt wird jedoch die Zugabe der Säure in Lösung.

Die benötigte Menge an Säure kann dabei an die betrieblichen Erfordernisse des Versuchs angepaßt werden. Zu beachten ist nur, daß nach Zugabe der Säure und Wasserstoffperoxid der pH-Wert des Lösung im gewünschten Bereich, wie oben beschrieben, liegt.

Bevorzugt liegt der Gehalt an freier Säure der abzureichernden Lösung im Bereich von 0,01 bis 25 Gew.-%, besonders bevorzugt im Bereich von 0,1 bis 10 Gew.-% und insbesondere im Bereich von 0,3 bis 5 Gew.-%.

Die Reihenfolge der Zugabe von Säure und Wasserstoffperoxid ist dabei im allgemeinen unkritisch. Denkbar ist beispielsweise, daß in die abzureichernde Lösung zuerst Säure gegeben wird und im Anschluß daran Wasserstoffperoxid. Ebenso ist die umgekehrte Reihenfolge möglich.

Das im Laufe des Verfahrens durch Oxidation gebildete Brom kann im erfindungsgemäßen Verfahren nach allen aus dem Stand der Technik bekannten Verfahren aus der Lösung entfernt werden.

Insbesondere kann der Abtreibvorgang durch Sieden oder Strippen der Lösung erfolgen. Ebenso denkbar ist das Anlegen von Vakuum an die Lösung.

Natürlich ist es auch denkbar, geeignete Verfahren zu kombinieren. So ist es beispielsweise möglich, die Lösung zu erhitzen und dabei gleichzeitig Vakuum anzulegen. Demgemäß betrifft die vorliegende Erfindung auch ein Verfahren, wie oben beschrieben, das dadurch gekennzeichnet ist, daß das während der Abreicherung entstehende Brom über Sieden der Lösung, Strippen der Lösung und/oder über Anlegen von Vakuum an die Lösung aus der Lösung entfernt wird.

Wird Brom durch alleiniges Strippen aus der Lösung entfernt, so wird Luft mit einem Durchsatz von im allgemeinen 1 bis 100, bevorzugt 2 bis 50, besonders bevorzugt von 3 bis 20 l/min in die Lösung eingeblasen.

Unabhängig von der Methode, die beim Abtreibvorgang verwendet wird, werden im erfindungsgemäßen Verfahren nach dem Abreichern Bromidgehalte gefunden, die bei < 500 ppm, bevorzugt bei < 300 ppm, besonders bevorzugt bei < 100 ppm, weiter besonders bevorzugt bei < 50 ppm, insbesondere bei < 25 ppm liegen.

Demgemäß betrifft die vorliegende Erfindung auch ein Verfahren, wie oben beschrieben, das dadurch gekennzeichnet ist, daß der Bromidgehalt der abgereicherten Lösung weniger als 25 ppm beträgt.

Ein weiterer Vorteil des erfindungsgemäßen Verfahrens liegt darin begründet, daß gleichzeitig mit Bromid ebenfalls, sofern vorhanden, Bromat aus der Lösung entfernt werden kann. Daher betrifft die vorliegende Erfindung auch ein Verfahren, wie oben beschrieben, das dadurch gekennzeichnet ist, daß zusätzlich zum Bromidgehalt der Bromatgehalt der Lösung abgereichert wird.

Nützlich ist Wasserstoffperoxid dabei besonders dann, wenn der Bromatgehalt der Lösung höher ist als deren Bromidgehalt.

In ihrer allgemeinsten Form betrifft die Erfindung die Verwendung von Wasserstoffperoxid zur Abreicherung von Bromid aus einer bromidhaltigen wäßrigen Lösung.

Weiter betrifft die vorliegende Erfindung die Verwendung von Wasserstoffperoxid zur Abreicherung von Bromat aus bromid- und bromathaltigen wäßrigen Lösungen.

Ebenso betrifft die vorliegende Erfindung die Verwendung von Wasserstoffperoxid zur Entfernung von Bromid oder Bromat oder einem Gemisch aus Bromid und Bromat aus einer wäßrigen Lösung, die bei der Herstellung von Arzneimitteln verwendet wird.

Im folgenden soll die vorliegende Erfindung durch ein Ausführungsbeispiel verdeutlicht werden.

### Beispiel:

Eine wäßrige Lösung enthielt 67 Gew.- % MgCl₂ × 6 H₂O, 0,093 Gew.- % Bromid und 2 Gew.- % an freier HCl.

Jeweils 1000 g dieser Originallösung wurden in zwei 2000 ccm-Bechergläser bzw. in einen 2000 ccm Kolben überführt.

Die Bechergläser wurden verwendet, um, nach der späteren Zugabe von Wasserstoffperoxid, das entstehende Brom durch Sieden bzw. Strippen auszutreiben und durch Absaugen einer geeigneten Absorption zugeführt.

Der Kolben wurde dazu verwendet, um nach Zugabe von Wasserstoffperoxid das entstehende Brom durch Anlegen von Vakuum auszutreiben.

Nach Zugaben von je 5 g 35 Gew.-%iger wäßriger Wasserstoffperoxidlösung zu jeder einzelnen Lösung wurden die Versuche gestartet. Die Lösungen in den beiden Bechergläsem wurden zum Sieden erhitzt bzw. durch Einblasen von Luft mit 4 l/min gestrippt. Dabei wurde das Gesamtvolumen der Lösung mittels Zugabe von Reinstwasser konstant gehalten.

Durch Anlegen von Vakuum (4 kPa, 30 Torr) an den Kolben wurde der dritte Versuch durchgeführt.

Unabhängig von der gewählten Austreibemethode wurden in allen drei Versuchen nach ionenchromatographischer Analyse Bromidgehalte von < 25 ppm gefunden.

## Patentansprüche

1. Verfahren zur Herstellung von Magnesiumchlorid mit einem Bromidgehalt von weniger als 500 ppm, **dadurch gekennzeichnet, daß** eine Lösung, enthaltend das Magnesiumchlorid, zur Bromidabreicherung mit Wasserstoffperoxid versetzt wird.

2. Verfahren nach Anspruch 1, **dadurch gekennzeichnet, daß** es sich um eine wäßrige Lösung handelt.

3. Verfahren nach Anspruch 1 oder 2, **dadurch gekennzeichnet, daß** die Lösung, aus der Bromid abgereichert wird, einen pH-Wert von ≤ 5 aufweist

4. Verfahren nach einem der Ansprüche 1 bis 3, **dadurch gekennzeichnet, daß** Magnesiumchlorid mit einem Bromidgehalt von weniger als 25 ppm hergestellt wird.

5. Verfahren nach einem der Ansprüche 1 bis 4, **dadurch gekennzeichnet, daß** zusätzlich zu Bromid aus der Lösung Bromat abgereichert wird.

## Claims

1. Method for preparing magnesium chloride having a bromide content of less than 500 ppm, **characterized in that**, for bromide depletion, hydrogen peroxide is added to a solution comprising the magnesium chloride.

2. Method according to Claim 1, **characterized in that** the solution is an aqueous solution.

3. Method according to Claim 1 or 2, **characterized in that** the solution from which bromide is depleted has a pH of ≤ 5.

4. Method according to one of Claims 1 to 3, **characterized in that** magnesium chloride having a bromide content of less than 25 ppm is prepared.

5. Method according to one of Claims 1 to 4, **characterized in that**, in addition to bromide, bromate is depleted from the solution.

## Revendications

1. Procédé de préparation de chlorure de magnésium avec une teneur en bromure inférieure à 500 ppm, **caractérisé en ce que** l'on mélange à du peroxyde d'hydrogène une solution contenant du chlorure de magnésium pour appauvrissement en bromure.

2. Procédé selon la revendication 1, **caractérisé en ce qu'**il s'agit d'une solution aqueuse.

3. Procédé selon la revendication 1 ou 2, **caractérisé en ce que** la solution que l'on appauvrit en bromure présente une valeur de pH ≤ 5.

4. Procédé selon l'une des revendications 1 à 3, **caractérisé en ce que** l'on produit le chlorure de magnésium avec une teneur en bromure inférieure à 25 ppm.

5. Procédé selon l'une des revendications 1 à 4, **caractérisé en ce qu'**outre le bromure on appauvrit la solution en bromate.
